(19) Europäisches Patentamt
European Patent Office
Office européen des brevets

(11) Veröffentlichungsnummer: **0 154 721**
**A1**

# (12) EUROPÄISCHE PATENTANMELDUNG

(21) Anmeldenummer: **84116345.4**

(22) Anmeldetag: **27.12.84**

(51) Int. Cl.⁴: **C 07 D 295/08**, C 07 D 285/10, C 07 D 417/12, C 07 D 249/14, A 61 K 31/40, A 61 K 31/41 // (C07D417/12, 285:10, 307:52),(C07D417/12, 285:10, 277:38)

(30) Priorität: **10.02.84 DE 3404786**

(43) Veröffentlichungstag der Anmeldung: **18.09.85 Patentblatt 85/38**

(84) Benannte Vertragsstaaten: **AT BE CH DE FR GB IT LI LU NL SE**

(71) Anmelder: **LUDWIG HEUMANN & CO GMBH, Heideloffstrasse 18-28 Postfach 2260, D-8500 Nürnberg (DE)**

(72) Erfinder: **Schickaneder, Helmut, Dr. Dipl.-Chem., Moosäcker 25, D-8501 Eckental (DE)**
Erfinder: **Postius, Stefan, Dr., Nunnenbeckstrasse 24, D-8500 Nürnberg 20 (DE)**
Erfinder: **Szelenyi, Istvan, Dr., Brahmsstrasse 16, D-8501 Schwaig (DE)**
Erfinder: **Mörsdorf, Peter, Dr. Dipl.-Chem., Untere Bahnhofstrasse 16, D-8501 Cadolzburg (DE)**
Erfinder: **Herter, Rolf, Dr. Dipl.-Chem., Bayernstrasse 42, D-8540 Schwabach (DE)**
Erfinder: **Ahrens, Kurt Henning, Dr., Praterstrasse 9, D-8500 Nürnberg (DE)**

(74) Vertreter: **Kraus, Walter, Dr. et al, Patentanwälte Kraus Weisert & Partner Thomas-Wimmer-Ring 15, D-8000 München 22 (DE)**

(54) **Neue Alkanolderivate, Verfahren zu ihrer Herstellung und diese Verbindungen enthaltendes Arzneimittel.**

(57) Es werden Alkanolderivate der allgemeinen Formel I

$$R^1R^2N-(CH_2)_m-Q-CH_2X-CH_2-Y-(CH_2)_n-NHR^3 \quad (I)$$

beschrieben, welche eine hohe selektive Wirkung auf Histamin-$H_2$-Rezeptoren besitzen. Diese Verbindungen können daher mit Vorteil für die Behandlung von durch erhöhte Magensäuresekretion hervorgerufene Krankheiten verwendet werden.

EP 0 154 721 A1

# BESCHREIBUNG

Die Erfindung betrifft neue Alkanolderivate mit einer hohen selektiven Wirkung auf Histamin-$H_2$-Rezeptoren, Verfahren zu ihrer Herstellung und Arzneimittel, die diese Verbindungen enthalten, sowie schließlich die Verwendung dieser Verbindungen in der Therapie.

Als Antiulcusmittel haben Cimetidin und Ranitidin bereits Eingang in die Therapie gefunden. Die Halbwertszeiten von Cimetidin und Ranitidin sind jedoch verhältnismäßig kurz. Dadurch wird es nötig, mehrfach täglich Tabletten mit Dosiseinheiten von 160 bis 300 mg in einer therapeutisch festgelegten Form zu verabreichen. Es besteht somit ein Bedarf an Antiulcusmitteln, die länger wirken und/oder wirksamer sind als Cimetidin und Ranitidin.

Bestimmte Verbindungen zeigen aufgrund ihrer spezfischen $H_2$-antagonistischen Aktivität eine Hemmung der Magensäuresekretion, wenn diese durch Histaminantagonisten stimuliert wird (Ash und Schild, "Brit. J. Pharmacol. Chemother.", 27, 427 (1966) und Black et al., "Nature", 236, 385 (1971)). Die pharmakologische Aktivität dieser Verbindungen, wie sie genauer weiter unten beschrieben werden, kann nach einer modifizierten Methode gemäß der DE-OS 27 34 070 beim perfundierten Rattenmagen gezeigt werden oder durch die Ermittlung der $pA_2$-Werte in vitro am Meerschweinchenvorhof (vgl. Ariens, Molecular Pharmacology, Band 1, Academic Press, New York, 1964) nachgewiesen werden. Weiterhin kann die $H_2$-antagonistische Wirkung an wachen Heidenhain-Pouch-Hunden nach der Methode von Black et al., "Nature", 236, 385 (1971) und an wachen Fistelkatzen gezeigt werden. Diese Verbindungen antagonisieren weiterhin die Histaminwirkung auf die Kontraktionsfrequenz des isolierten rechten Atriums des Meerschweinchens, aber sie beeinflussen nicht histamininduzierte Kontraktionen des isolierten, glatten gastrointestinalen Muskels, wenn diese durch $H_2$-Agonisten hervorgerufen werden.

Nachdem Hemmstoffe für Histamin-$H_2$-Rezeptoren sowohl bezüglich der basalen Magensäuresekretion als auch der durch Gastrin, Histamin,

Methacholin oder Nahrung induzierten Magensäuresekretion Hemmwirkung besitzen, können sie für die Behandlung von peptischen Ulcera, die durch übermäßige Sekretion von Magensäure verursacht sind, und bei der Therapie hyperacider Gastritis verwendet werden.

Der Erfindung liegt die Aufgabe zugrunde, neue Hemmstoffe für Histamin-$H_2$-Rezeptoren mit verbesserter und/oder länger anhaltender Wirksamkeit zur Verfügung zu stellen.

Diese Aufgabe wird durch die Erfindung gelöst.

Gegenstand der Erfindung sind daher neue Alkanolderivate der allgemeinen Formel I

$$R^1R^2N-(CH_2)_m-Q-CH_2X-CH_2-Y-(CH_2)_n-NHR^3 \qquad (I)$$

in der im Falle m = 1 $R^1$ und $R^2$, die gleich oder verschieden sein können, jeweils für Wasserstoff, $C_{1-10}$-Alkyl, $C_{5-6}$-Cycloalkyl, Amino, Niedrigalkylamino, Niedrigdialkylamino oder im Falle m = 0 für $(NH_2)_2C=$ stehen oder $R^1$ und $R^2$ zusammen mit dem Stickstoffatom, an das sie gebunden sind, einen 5- bis 8gliedrigen alicyclischen unsubstituierten oder mit einer Methylgruppe substituierten heterocyclischen Ring bilden, m für 0 oder 1 steht und Q einen Furan-, Thiophen-, Thiazol- oder Benzolring bedeutet, X für ein Schwefel- oder Sauerstoffatom oder für die Gruppe -CHOH steht, Y eine Einfachbindung oder die Gruppe -CHOH darstellt, n 1 oder 2 bedeutet und $R^3$ für die Gruppierung

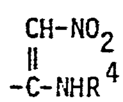 , 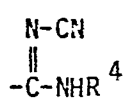 , 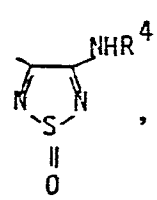 , 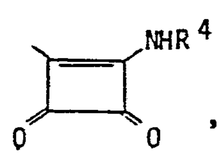 ,

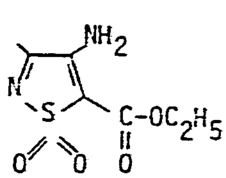 oder 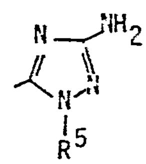

steht, worin $R^4$ ein Wasserstoffatom, eine $C_{1-3}$-Alkylgruppe oder eine Propargylgruppe, $R^5$ ein Wasserstoffatom oder eine $C_{1-3}$-Alkylgruppe bedeutet, sowie ihre physiologisch annehmbaren Salze und Hydrate.

In der allgemeinen Formel I bedeuten $R^1$ und $R^2$, die gleich oder verschieden sein können, Wasserstoff, $C_{1-10}$-Alkyl, vorzugsweise $C_{1-3}$-Alkyl (Niedrigalkyl), $C_{5-6}$-Cycloalkyl, wie Cyclopentyl und Cyclohexyl, Amino, Niedrigalkylamino, wie Methyl-, Ethyl- oder Propylamino, im Falle, daß m den Wert 1 hat. Wenn m den Wert 0 hat, dann bilden $R^1$ und $R^2$ miteinander die Gruppierung $(NH_2)_2C=$. $R^1$ und $R^2$ können auch mit dem Stickstoffatom, an das sie gebunden sind, einen 5- bis 8gliedrigen unsubstituierten oder mit einer Methylgruppe substituierten heterocyclischen Ring bilden. Als Beispiele für solche Ringe können der Pyrrolidin-, Methylpyrrolidin-, Morpholin-, Thiomorpholin-, Piperidin-, Methylpiperidin-, N-Methylpiperazin-, Homopiperidin-, Heptamethylenimino- und Octamethyleniminoring genannt werden, wobei der Pyrrolidin- oder Piperidinring bevorzugt werden.

Q bedeutet einen Furan-, Thiophen-, Thiazol- oder Benzolring. Der Furanring ist vorzugsweise in 2,5-Stellung in das Molekül eingefügt. Der Thiophenring ist vorzugsweise in 2,5- oder 2,4-Stellung eingefügt, während der Thiazolring vorzugsweise in 2,4-Stellung eingefügt ist. Der Benzolring ist vorzugsweise durch Bindungen in 1- und 3- oder 1- und 4-Stellung eingefügt. Von diesen Bausteinen wird der Benzolring bevorzugt, wobei ein in 1- und 3-Stellung eingearbeiteter Benzolring ganz besonders bevorzugt wird.

X steht für ein Schwefel- oder Sauerstoffatom oder für die Gruppe -CHOH. Y bedeutet eine Einfachbindung oder die Gruppe -CHOH. n kann die Werte 1 und 2 einnehmen. $R^3$ steht für eine der folgenden Gruppen:

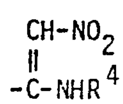 ,
(a)

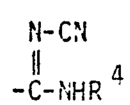 ,
(b)

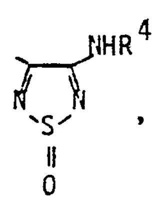 ,
(c)

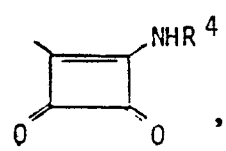 ,
(d)

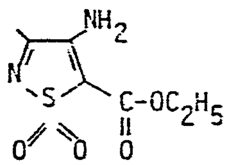

oder

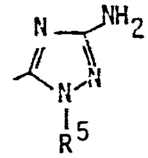

(e) (f)

Von diesen Bausteinen werden die Gruppierungen (c), (d) und (e) bevorzugt.

In diesen Gruppierungen bedeutet $R^4$ ein Wasserstoffatom, eine $C_{1-3}$-Alkylgruppe (Niedrigalkylgruppe) oder eine Propargylgruppe. $R^5$ steht für ein Wasserstoffatom oder eine $C_{1-3}$-Alkylgruppe (Niedrigalkylgruppe).

Vorzugsweise bedeuten, wenn in der allgemeinen Formel I Q für einen durch Bindungen in 2- und 4-Stellung eingefügten Thiazolring steht, m = 0, $R^1$ und $R^2$ $(NH_2)_2C=$, X ein Schwefelatom, Y die Gruppe -CHOH, n = 1, $R^3$ $-\overset{\overset{N-CN}{\|}}{C}-NHR^4$ oder 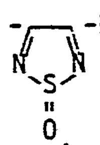 -$NHR^4$

und $R^4$ ein Wasserstoffatom, eine Methyl- oder Propargylgruppe.

Vorzugsweise bedeuten, wenn in der allgemeinen Formel I Q für einen durch Bindungen in 2- und 5-Stellung eingefügten Furanring oder einen in 2- und 5- oder 2- und 4-Stellungen eingefügten Thiophenring steht, m = 1 und $R^1$ ein Wasserstoffatom. In diesem Fall wird weiterhin bevorzugt, daß $R^1$ $C_{1-10}$-Alkyl, insbesondere $C_{1-3}$-Alkyl, ist und $R^2$ $C_{5-6}$-Cycloalkyl ist.

Eine bevorzugte Gruppe von erfindungsgemäßen Verbindungen ist dadurch gekennzeichnet, daß $R^1$ und $R^2$ für Methyl und/oder Ethyl stehen oder $R^1$ und $R^2$ zusammen mit dem Stickstoffatom, an das sie gebunden sind, einen substituierten oder unsubstituierten 5- bis 6gliedrigen alicyclischen heterocyclischen Ring, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring, insbesondere einen Pyrrolidin- oder Piperidinring, bilden.

Eine besonders bevorzugte Gruppe von erfindungsgemäßen Verbindungen ist dadurch gekennzeichnet, daß $R^1$ und $R^2$ zusammen mit dem Stickstoffatom, an das sie gebunden sind, einen substituierten oder unsubstituierten 5- bis 8gliedrigen alicyclischen heterocyclischen Ring, wie oben beschrieben, insbesondere einen Pyrrolidin- oder Piperidinring, bilden.

Eine weiterhin bevorzugte Gruppe von erfindungsgemäßen Verbindungen ist dadurch gekennzeichnet, daß $R^1$, $R^2$ und m die oben genannten Bedeutungen haben, Q für einen Benzolring steht, dessen Einfügung in den Rest des Moleküls durch Bindung in 1- und 3- oder 1- und 4-Stellung, vorzugsweise in 1- und 3-Stellung, erfolgt, X die Gruppierung -CHOH, Y eine Einfachbindung und n 1 oder 2 bedeutet und $R^3$ für die in Anspruch 1 angegebenen Reste, wie oben beschrieben, steht.

Die erfindungsgemäßen Verbindungen werden wie folgt hergestellt:

a) Zur Herstellung von Verbindungen, bei denen $R^3$ für

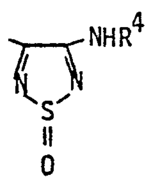
oder
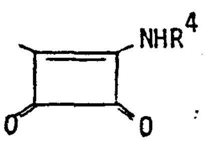

steht, wird eine Verbindung der allgemeinen Formel II

$$R^1R^2N(CH_2)_m\text{-}Q\text{-}CH_2\text{-}X\text{-}CH_2\text{-}Y\text{-}(CH_2)_nNH\text{-}R^6 \qquad (II)$$

in der $R^1$, $R^2$, m, Q, X, Y und n die oben angegebenen Bedeutungen besitzten und $R^6$ für die Gruppe

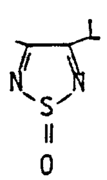
oder
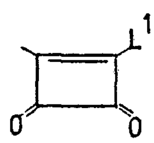

steht, wobei $L^1$ eine Methoxy-, Ethoxy- oder Butoxygruppe als Austrittsgruppe bedeutet, in einem Lösungsmittel mit einem Amin der allgemeinen Formel III

$$R^4NH_2 \qquad (III)$$

in der $R^4$ für ein Wasserstoffatom, eine Methylgruppe oder eine Propargylgruppe steht, zu der erfindungsgemäßen Verbindung der allgemeinen Formel I umgesetzt. Die Umsetzung findet in einem Lösungsmittel, beispielsweise in einer alkoholischen Lösung, wie von Methanol, Ethanol oder Isopropanol, bevorzugt Ethanol, statt. Vorzugsweise wird mit äquimolaren Mengen der Verbindung der allgemeinen Formel II und des Amins der allgemeinen Formel III gearbeitet. Die Reaktionstemperatur ist Raumtemperatur bis Rückflußtemperatur. Die Aufarbeitung und Isolierung erfolgt in üblicher Weise. Die erhaltene Verbindung kann gegebenenfalls in ihr physiologisch annehmbares Salz umgewandelt werden.

b) Zur Herstellung von Verbindungen, bei denen $R^3$ für $\overset{CH-NO_2}{\overset{\|}{-C}-NHR^4}$ oder $\overset{N-CN}{\overset{\|}{-C}-NHR^4}$ steht, wird eine Verbindung der allgemeinen Formel IV

$$R^1R^2N(CH_2)_m-Q-CH_2-X-CH_2-Y-(CH_2)_n-NHR^7 \qquad (IV)$$

in der $R^1$, $R^2$, m, Q, X, Y und n die oben angegebenen Bedeutungen haben und $R^7$ für $\overset{CH-NO_2}{\overset{\|}{C}-L^2}$ oder $\overset{N-CN}{\overset{\|}{-C}-L^2}$ steht, wobei $L^2$ für eine Thiomethyl-, Methoxy-, Ethoxy- oder Phenoxygruppe als Austrittsgruppe steht, mit einem Amin der allgemeinen Formel III

$$R^4NH_2 \qquad (III)$$

zu einer erfindungsgemäßen Verbindung der allgemeinen Formel I umgesetzt. Die Umsetzung erfolgt vorzugsweise in einem Lösungsmittel, wie einer alkoholischen Lösung, wie zum Beispiel von Methanol, Ethanol oder Isopropanol, bevorzugt Ethanol. Die Reaktanten können beispielsweise in äquimolaren Mengen eingesetzt werden. Die Reaktionstemperatur ist Raumtemperatur bis Rückflußtemperatur des verwendeten Lösungsmittels. Die Aufarbeitung und Isolierung erfolgt in üblicher Weise. Die erhaltene Verbindung kann gegebenenfalls in ihr physiologisch annehmbares Salz umgewandelt werden.

c) Zur Herstellung von Verbindungen, bei denen $R^3$ für

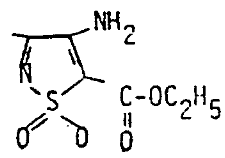

steht, wird ein Amin der allgemeinen Formel V

$$R^1R^2N(CH_2)_m-Q-CH_2-X-CH_2-Y-(CH_2)_n-NH_2 \qquad (V)$$

in der $R^1$, $R^2$, m, Q, X, Y und n die oben angegebenen Bedeutungen haben, mit einer Verbindung der Formel VI

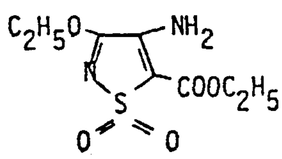

(VI)

zu einer erfindungsgemäßen Verbindung der allgemeinen Formel I umgesetzt. Auch in diesem Falle wird die Reaktion vorzugsweise in einem Lösungsmittel, beispielsweise in einem Alkohol, wie Methanol, Ethanol oder Isopropanol, bevorzugt Ethanol, und unter Anwendung von äquimolaren Mengen der Reaktanten durchgeführt. Die Reaktionstemperatur ist Raumtemperatur bis Rückflußtemperatur des Lösungsmittels. Die bei dieser Reaktion eingesetzte Verbindung der Formel VI ist bekannt und wird beispielsweise in J. Org. Chem. 48, 763 (1983) beschrieben. Die Aufarbeitung und Isolierung erfolgt in üblicher Weise. Die erhaltene Verbindung kann gegebenenfalls in ihr physiologisch annehmbares Salz umgewandelt werden.

d) Zur Herstellung von Verbindungen, bei denen $R^3$ für 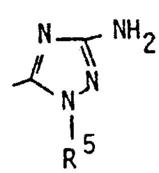

steht und $R^5$ für ein Wasserstoffatom, eine Methyl- oder Ethylgruppe steht, wird eine Verbindung der allgemeinen Formel VII

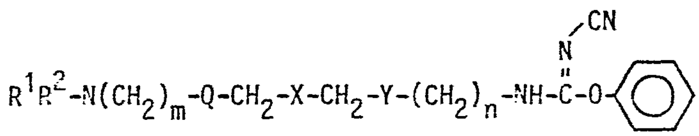 (VII)

in der $R^1$, $R^2$, m, Q, X, Y und n die oben angegebenen Bedeutungen haben, mit Hydrazinhydrat, Methylhydrazin oder Ethylhydrazin in einem Lösungsmittel zu einer erfindungsgemäßen Verbindung der allgemeinen Formel I umgesetzt. Auch hier erfolgt vorzugsweise die Umsetzung in einem Lösungsmittel, beispielsweise einem Alkohol, wie Methanol, Ethanol oder Isopropanol, bevorzugt Ethanol. Die Umsetzung kann bei Raumtemperatur bis Rückflußtemperatur des verwendeten Lösungsmittels und beispielsweise unter Anwendung äquimolarer Mengen durchgeführt werden. Die Aufarbeitung und Isolierung erfolgt in üblicher Weise. Die erhaltene Verbindung kann gegebenenfalls in ihr physiologisch annehmbares Salz umgewandelt werden.

Die Erfindung umfaßt auch die Verbindungen der Formel I in Form ihrer verschiedenen stereochemischen Formen (Enantiomeren), ihrer physiologisch annehmbaren Hydrate und Salze mit anorganischen und organischen Säuren. Diese Salze können zum Beispiel mit Mineralsäure, wie Chlor-, Brom- und Jodwasserstoffsäure, Phosphorsäure, Metaphosphorsäure, Salpetersäure oder Schwefelsäure oder mit organischen Säuren, wie Ameisensäure, Essigsäure, Propionsäure, Phenylessigsäure, Weinsäure, Zitronensäure, Fumarsäure, Methansulfonsäure etc. gebildet werden.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmazeutisch annehmbaren Trägern oder Verdünnungsmitteln hergestellt werden.

Die erfindungsgemäßen Verbindungen können daher für die orale, bukkale, topische, parenterale oder rektale Verabreichung formuliert werden, wobei die orale Verabreichung bevorzugt wird. Für die orale Verabreichung kann das Arzneimittel in Form von beispielsweise Tabletten, Kapseln,

Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, vorzugsweise 5 bis 250 mg/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem

Zeitpunkt bzw. Intervall, zu welchem die Verabreichung erfolgt. So gibt es zum Beispiel Fälle, wo mit weniger als der oben genannten Mindestemenge ausgekommen werden kann, während in anderen Fällen die genannte obere Grenze überschritten werden muß.

Die erfindungsgemäßen Verbindungen zeichnen sich gegenüber anerkannt guten Arzneimitteln der gleichen Wirkungsrichtung durch eine Verbesserung der pharmakologischen Aktivitäten aus. Dies ergibt sich aus den Ergebnissen der im folgenden dargestellten pharmakologischen Vergleichsuntersuchungen.

Versuchsmodell: Gosh-Schild-Ratte
(Stimulator: Histamin)

| | | |
|---|---|---|
| Cimetidin (Vergleich) | i.V. $ID_{50}$ | 1,4 µmol/kg |
| Beispiel 1 | i.V. $ID_{50}$ | 0,075 µmol/kg |

Beispiel 1

a) Herstellung von

2-[2-Hydroxy-3-[3-(1-piperidylmethyl)benzyloxy]propyl]-1H-isoindol-1,3-dion

Eine Mischung aus 10.25 g (0.05 mol) 3-(1-Piperidylmethyl)-benzylalkohol und 10.15 g (0.05 mol) N-(2,3-Epoxypropyl)phthalimid wird 80 Minuten bei 130 °C unter Stickstoff gerührt. Das erhaltene, zähe Harz wird mit Methylenchlorid/Methanol 9 : 1 an Kieselgel chromatographiert. Die Hauptfraktion ergibt nach Eindampfen 8.60 g (42 %) der Titelverbindung als hellbraunes Öl.

b) Herstellung von

2-Hydroxy-3-[3-(1-piperidylmethyl)benzyloxy]propylamin

8.60 g (0,021 mol) 2-[2-Hydroxy-3-[3-(1-piperidylmethyl)benzyloxy]propyl]-1H-isoindol-1,3-dion und 3.3 ml Hydrazinhydrat (80 %) werden in 80 ml Ethanol 3 Stunden gekocht. Der nach Eindampfen der Mischung verbleibende Rückstand wird in 50 ml Wasser aufgenommen, mit 8 ml konz. Salzsäure versetzt und filtriert. Das Filtrat wird mit konz. Natronlauge auf pH 12 eingestellt und mit 3 x 40 ml Methylenchlorid extrahiert. Die organische Phase wird mit $Na_2SO_4$ getrocknet und im Vakuum eingedampft. Der verbleibende Rückstand wird im Hochvakuum destilliert. Man erhält 4.44 g (76 %) eines farblosen Öls vom Sdp. 145 - 155 ° / $1 \times 10^{-2}$ mbar.

c) Herstellung von

3-[2-Hydroxy-3-[3-(1-piperidylmethyl)benzyloxy]propylamino]-4-ethoxy-1,2,5-thiadiazol-1-oxid

Eine Lösung von 2.78 g (0.01 mol) 2-Hydroxy-3-[3-(1-piperidylmethyl)benzyloxy]propylamin in 10 ml Ethanol wird während 30 Minuten zu einer Lösung von 1.90 g (0.01 mol) 3,4-Diethoxy-1,2,5-thiadiazol-1-oxid in 10 ml Ethanol getropft und die Mischung 4 Stunden bei Raumtemperatur gerührt. Nach dem Einengen der Reaktionslösung erhält man die Titelverbindung, die ohne weitere Reinigung umgesetzt wird.

d) Herstellung von

3-[2-Hydroxy-3-[3-(1-piperidylmethyl)-benzyloxy]propylamino]-4-amino-1,2,5-thiadiazol-1-oxid

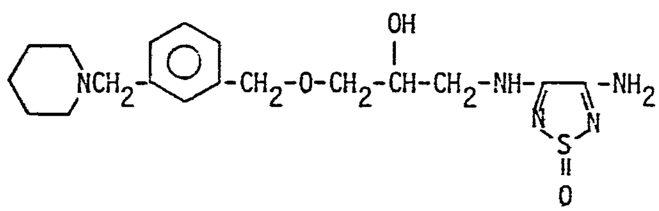

Nach Zugabe von 20 ml ethanolischem Ammoniak (5 mol/l) zu der o.g. eingeengten Reaktionslösung wird über Nacht weitergerührt. Der nach Eindampfen der Lösung erhaltene farblose Feststoff wird mit Methanol/konz. Ammoniak 99 : 1 an Kieselgel chromatographiert und gibt nach Eindampfen der Hauptfraktion 2.80 g (71 %) der Titelverbindung.

Farblose Kristalle vom Schmelzpunkt 109,6 - 111 °C

Rf = 0.49 ($CH_3OH/NH_3$ konz. 99 : 1 )

| | | | | |
|---|---|---|---|---|
| $C_{18}H_{27}N_5O_3S$ (393.5) | Ber.: | C 54.94 | H 6.92 | N 17.80 |
| | Gef.: | C 54.87 | H 6.94 | N 17.78 |

| | | | |
|---|---|---|---|
| $^1$H-NMR-Spektrum: | $\delta$ = 1.25 - 1.70 (m) | 6 H, | |
| ($d_6$-DMSO, TMS als | 2.21 - 2.46 (m) | 4 H, | |
| interner Standard) | 3.23 - 3.75 (m) | 4 H, | |
| | 3.40 (s) | 2 H, | |
| | 3.93 (m) | 1 H, | |
| | 4.49 (s) | 2 H, | |
| | 5.21 (m, breit) | 1 H, | austauschbar mit $D_2O$ |
| | 7.27 (s) | 4 H, | |
| | 8.1 (m) | 3 H, | austauschbar mit $D_2O$ |

Beispiel 2

a) Herstellung von

1-[2-Hydroxy-3-[3-(1-piperidylmethyl)benzyloxy]propylamino]-2-ethoxy-cyclobuten-3,4-dion

2.78 g (0.01 mol) 2-Hydroxy-3-[3-(1-piperidylmethyl)benzyloxy]propylamin in 10 ml Ethanol werden langsam zu 1.70 g (0.01 mol) 1,2-Diethoxy-cyclobuten-3,4-dion getropft und die Mischung 5 Stunden gerührt. Nach dem Einengen der Reaktionslösung erhält man die Titelverbindung, die ohne weitere Reinigung umgesetzt wird.

b) Herstellung von

1-[2-Hydroxy-3-[3-(1-piperidylmethyl)benzyloxy]propylamino]-2-amino-cyclobuten-3,4-dion

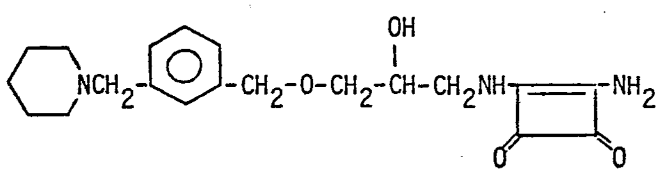

Nach Zugabe von 20 ml ethanolischem Ammoniak zu der o. g. eingeengten Reaktionslösung fällt ein schwachgelber Niederschlag, der nach 18 Stunden abgesaugt und chromatographisch mit Methanol an Kieselgel gereinigt wird.

Ausbeute: 1.86 g (50 %)

Farblose Kristalle vom Schmelzpunkt 201 - 203 °C (zers.)

Rf = 0.58 ($CH_3OH/NH_3$ konz. 99 : 1)

$C_{20}H_{27}N_3O_4$ (373,5)

$^1$H-NMR-Spektrum: ($d_6$-DMSO, TMS als interner Standard)

δ = 1.28 - 1.62 (m) 6 H,
2.19 - 2.42 (m) 4 H,
3.25 - 3.93 (m) 5 H,
3.40 (s) 2 H,
4.48 (s) 2 H,
5.22 (breit) 1 H, austauschbar mit $D_2O$
7.24 (s) 4 H,
7.44 (breit) 3 H, austauschbar mit $D_2O$

Beispiel 3

a) Herstellung von

$N^1$-Cyano-$N^2$-[2-hydroxy-3-[3-(1-piperidylmethyl)benzyloxy]propyl]-S-methyl-isothioharnstoff

Zu einer Lösung von 0.73 g (5 mmol) N-Cyan-dimethyldithiocarbonat in 15 ml Diethylether werden langsam 1.39 g (5 mmol) 2-Hydroxy-3-[3-(1-piperidyl-methyl)benzyloxy]propylamin in 25 ml Diethylether und 5 ml Methanol getropft. Nach 24-stündigem Rühren bei Raumtemperatur wird die Lösung im Vakuum eingedampft. Man erhält 1.9 g der Titelverbindung in Form eines farblosen zähen Öls.

b) Herstellung von

3-Amino-5-[2-hydroxy-3-[3-(1-piperidylmethyl)benzyloxy]-propylamino]-1-methyl-1H-1,2,4-triazol

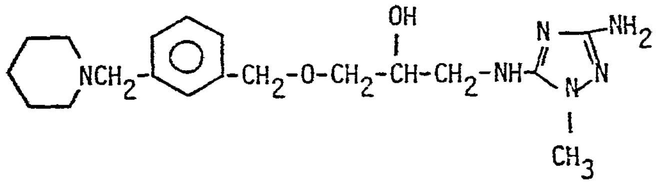

0.46 g (10 mmol) Methylhydrazin werden zu einer Lösung von 1.9 g (5 mmol) $N^1$-Cyano-$N^2$-[2-hydroxy-3-[3-(1-piperidylmethyl)benzyloxy]propyl]-S-methyl-isothioharnstoff in 10 ml Ethanol gegeben. Nach 24 Stunden bei Raumtemperatur wird das Lösungsmittel im Vakuum abgedampft und der Rückstand mit Methylenchlorid/ Methanol 1 : 1 an Kieselgel gereinigt. Nach Abdampfen des Lösungsmittels wird die Titelverbindung als amorpher Feststoff erhalten.

Ausbeute: 0.82 g (44 %)

Rf = 0.55 ($CH_3OH$/konz. $NH_3$ 99:1)

$C_{19}H_{30}N_6O_2$ (374.5)

| | |
|---|---|
| $^1$H-NMR-Spektrum: (CDCl$_3$, TMS als interner Standard) | $\delta$ = 1.31 - 1.73 (m) 6 H, |
| | 2.23 - 2.52 (m) 4 H, |
| | 3.26 (s) 3 H, |
| | 3.44 (s) 2 H, |
| | 3.32 - 3.71 (m) 4 H, |
| | 3.87 - 4.23 (m) 3 H, 2 H austauschbar mit $D_2O$ |
| | 4.50 (s) 2 H, |
| | 4.77 (t, breit) 1 H, austauschbar mit $D_2O$ |
| | ~4.8 (breit) 1 H, austauschbar mit $D_2O$ |
| | 7.14 - 7.37 (s, breit) 4 H. |

## Beispiel 4

a) Herstellung von

<u>1-Chlor-3-dibenzylamino-2-propanol</u>

49.3 g (0.25 mol) Dibenzylamin und 25.4 g (0.275 mol) Epichlorhydrin werden 3 Stunden unter Stickstoff bei 85 - 90 °C gerührt. Das erhaltene goldgelbe Öl wird im Hochvakuum destilliert. Man erhält 50.5 g (70 %) eines farblosen Öls.

Sdp. 150 - 155 °C ($1.5 \times 10^{-2}$ mbar)

b) Herstellung von

2-Dibenzylaminomethyl-oxiran

50.7 g (0.175 mol) 1-Chlor-3-dibenzylamino-2-propanol werden mit 9.5 g (0.24 Mol) Natriumhydroxid und 5 ml Wasser 1 Stunde bei 95° C gerührt. Nach Zugabe von 50 ml Chloroform und 20 ml Wasser werden die Phasen getrennt, die organische Phase wird mit 20 ml Wasser gewaschen, mit Natriumsulfat getrocknet und eingedampft. Die Destillation des erhaltenen gelben Öls im Vakuum ergibt 33.7 g (76 %) des Oxirans als farbloses Öl.

Sdp. 125 ° C (1.5 x $10^{-2}$ mbar)

c) Herstellung von

1-Dibenzylamino-3-[3-(1-piperidylmethyl)phenyl]-2-propanol

Zu 0.97 g (0.04 mol) Magnesium-Spänen in 5 ml Tetrahydrofuran werden bei 60 ° C Innentemperatur 10.2 g (0.04 mol) 3-(1-Piperidylmethyl)-brombenzol in 20 ml Tetrahydrofuran getropft. Nach beendeter Zugabe wird 30 Minuten bei 60 ° C gerührt, dann wird die Lösung auf 10° C gekühlt. 10.2 g (0.04 mol) 2-Dibenzylaminomethyl-oxiran in 20 ml Tetrahydrofuran werden langsam zugetropft und die erhaltene Lösung wird 2 Stunden bei Raumtemperatur gerüht. Nach Zugabe von 20 ml Eiswasser und 4.5 g Ammoniumchlorid wird die wäßrige Phase abgetrennt und mit 25 ml Methylenchlorid extrahiert. Die vereinigten organischen Phasen ergeben nach Trocknen mit Natriumsulfat und Einengen ein gelbes Harz, das mit Methylenchlorid/Methanol (9:1) an Kieselgel chromatographiert wird. Die 2. Fraktion liefert nach Einengen die Titelverbindung als schwachgelbes Öl.

Ausbeute: 11.5 g (67 %)

d) Herstellung von

1-Amino-3-[3-(1-piperidylmethyl)phenyl]-2-propanol

11.5 g (0.027 mol) 1-Dibenzylamino-3-[3-(1-piperidylmethyl)phenyl]-2-propanol in 90 ml Ethanol und 10 ml Wasser werden in Gegenwart von 0.5 g Palladium/Aktivkohle (10 % Pd) bei 35 ° C und Atmosphärendruck hydriert.

Nach Abfiltrieren des Katalysators und Abdampfen des Lösungsmittels verbleiben 5.8 g eines farblosen Öls, das nach chromatographischer Reinigung mit Methanol/Ammoniak konz. (95:5) 3.5 g (52 %) der Titelverbindung in Form eines farblosen Öls ergibt.

e) Herstellung von

3-[2-Hydroxy-3-[3-(1-piperidylmethyl)phenyl]propylamino]-4-amino-1,2,5-thiadiazol-1-oxid

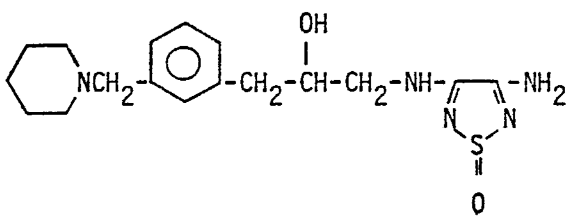

Analog Beispiel 1 c,d werden aus 0.47 g (1.9 mmol) 1-Amino-3-[3-(1-piperidylmethyl)phenyl]-2-propanol und 0.36 g (1.9 mmol) 3,4-Diethoxy-1,2,5-thiadiazol-1-oxid nach Chromatographie an Kieselgel mit Methanol/konz. Ammoniak (95 : 5) als Eluent 0.52 g (76 %) der Titelverbindung erhalten.

Farbloser Feststoff, Schmelzpunkt 94 - 96 °C

Rf = 0.55 ($CH_3OH/NH_3$ konz. 95:5)

$C_{17}H_{25}N_5O_2S$ (363.5)

$^1$H-NMR-Spektrum: (d$_6$-DMSO, TMS als interner Standard)

$\delta$ = 1.22 - 1.65 (m) 6 H,
2.20 - 2.47 (m) 4 H,
2.73 (d, breit) 2 H,
3.00 - 3.78 (m) 3 H,
3.41 (s) 2 H,
3.94 (m) 1 H,
5.53 (breit) 1 H, austauschbar mit $D_2O$
7.04 - 7.34 (m) 4 H,
8.05 , 8.29 (breit) 2 H, austauschbar mit $D_2O$

Beispiel 5

a) Herstellung von

2-Hydroxy-3-[3-(1-piperidylmethyl)phenyl]-butyronitril

0.74 g (3.2 mmol) 2-[3-(1-Piperidylmethyl)phenyl]methyl-oxiran, 0.294 g (6 mmol) Natriumcyanid und 0.107 g (2 mmol) Ammoniumchlorid werden in 5 ml Ethanol/ 5 ml Wasser 6 Stunden gekocht. Nach weitgehendem Einengen der Mutterlauge im Vakuum wird der Rückstand in 10 ml Wasser aufgenommen, die Lösung mit Kaliumcarbonat auf pH 12 eingestellt und mit 3 x 20 ml Methylenchlorid extrahiert. Die organische Phase ergibt nach Trocknen und Eindampfen im Vakuum 0.82 g eines braunen Öls.

b) Herstellung von

4-Amino-1-[3-(1-piperidylmethyl) phenyl]-2-butanol

Zu 0.82 g (3.2 mmol) 2-Hydroxy-3-[3-(1-piperidylmethyl)phenyl]-butyronitril in 30 ml Ether und 10 ml Tetrahydrofuran werden 0.10 g (2.6 mmol) Lithiumaluminiumhydrid gegeben und die Mischung 2 Stunden zum Rückfluß erhitzt. Nach Zugabe von 0.25 ml Wasser wird der entstandene Niederschlag abgesaugt, mit 20 ml Methylenchlorid aufgeschlämmt und nochmals abgesaugt. Nach Trocknen der vereinigten organischen Phasen und Eindampfen im Vakuum verbleiben 0.65 g eines gelben Öls, die im Hochvakuum destilliert 0.51 g (61 %) der Titelverbindung ergeben.

Farbloses, zähes Öl, Sdp. 140 - 150 $^{o}$ C / $7x10^{-3}$ mbar.

c) Herstellung von

3-[3-Hydroxy-4-[3-(1-piperidylmethyl)phenyl] butyl]amino-4-amino-1,2,5-thiadiazol-1-oxid

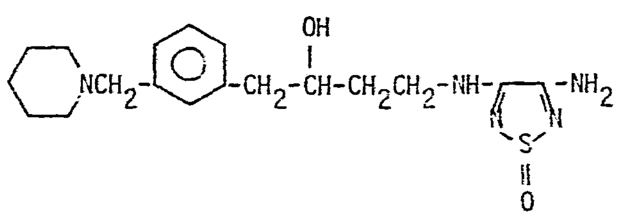

0.51 g (1.95 mmol) 4-Amino-1-[3-(1-piperidylmethyl)phenyl]-2-butanol in 5 ml Ethanol werden analog Beispiel 1,c zu 0.37 g (1.95 mmol) 3,4-Diethoxy-1,2,5-thiadiazol-1-oxid in 5 ml Ethanol getropft. Nach 4 Stunden bei Raumtemperatur werden 6 ml ethanol. Ammoniak (5 mol/l) zugesetzt und die Lösung über Nacht gerührt. Der nach Abdampfen des Lösungsmittels im Vakuum erhaltene Feststoff wird mit Methanol an Kieselgel chromatographiert.

Ausbeute : 0.57 g (77 %)

Farbloser Feststoff vom Schmelzpunkt 74 - 77 °C

Rf = 0.47 ($CH_3OH/NH_3$ konz. 99 : 1)

$C_{18}H_{27}N_5O_2S$ (377.5)

| | | | |
|---|---|---|---|
| $^1$H-NMR-Spektrum: | δ = | 1.28 - 1.80 (m) | 8 H, |
| ($d_6$-DMSO, TMS als | | 2.19 - 2.42 (m) | 4 H, |
| interner Standard) | | 2.69 (d, breit) | 2 H, |
| | | 3.23 - 3.92 (m) | 4 H, |
| | | 3.38 (s) | 2 H, |
| | | 4.70 (breit) | 1 H, austauschbar mit $D_2O$ |
| | | 7.01 - 7.35 (m) | 4 H, |
| | | 7.95 (breit) | 2 H, austauschbar mit $D_2O$ |

Beispiel 6

Herstellung von

3-[2-Hydroxy-3-[3-(1-piperidylmethyl)benzyloxy]propylamino]-4-amino-5-ethoxycarbonyl-isothiazol-1,1-dioxid

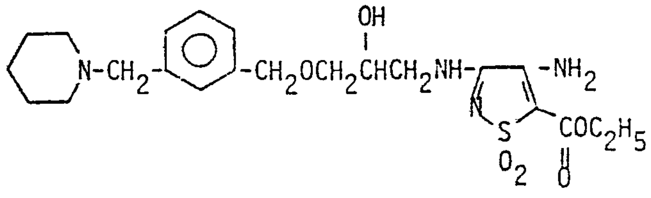

1.39 g (5 mmol) 2-Hydroxy-3-[3-(1-piperidylmethyl)benzyloxy]propylamin werden zur Suspension von 1.24 g (5 mmol) 4-Amino-3-ethoxy-5-ethoxycarbonyl-isothiazol-1,1-dioxid in 10 ml Acetonitril getropft und die Mischung 5 Stunden bei Raumtemperatur gerührt. Der nach Abdampfen des Lösungsmittels i. Vak. erhaltene Rückstand wird mit Methanol an Kieselgel chromatographiert. Die Hauptfraktion ergibt 2,06 g (86 %) der Titelverbindung.

Farbloser amorpher Feststoff vom Schmelzpunkt 72 - 74 °C

Rf = 0.45 ($CH_3OH/NH_3$ konz. 99 : 1 )

$C_{22}H_{32}N_4O_6S$ (480,6)

| | | |
|---|---|---|
| $^1$H-NMR-Spektrum: | $\delta$ = 1.25 (t) | 3 H, |
| ($d_6$-DMSO, TMS als | 1.30 - 1.66 (m) | 6 H, |
| interner Standard) | 2.21 - 2.47 (m) | 4 H, |
| | 3.20 - 3.77 (m) | 4 H, |
| | 3.44 (s) | 2 H, |
| | 3.93 (m) | 1 H, |
| | 4.23 (q) | 2 H, |
| | 4.51 (s) | 2 H, |
| | 7.28 (s) | 4 H, |
| | ~8.2 (breit) | 3 H, austauschbar mit $D_2O$ |

Beispiel 7

Herstellung von

<u>N[1]-Cyano-N[2]-methyl-N[3]-[2-hydroxy-3-[3-(1-piperidylmethyl)benzyloxy]propyl]-guanidin</u>

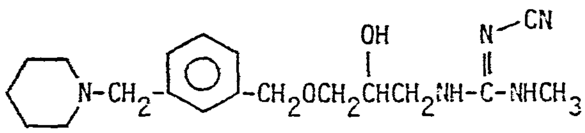

In eine Lösung von 1.9 g (5 mmol) $N^1$-Cyano-$N^2$-[2-hydroxy-3-[3-(1-piperidylmethyl)benzyloxy]propyl]-S-methyl-isothioharnstoff in 30 ml Ethanol werden unter Eiskühlung bei 0 bis + 3 °C 2,0 g (65 mmol) Methylamin eingeleitet. Nach 2-stündigem Rühren bei Raumtemperatur wird das Lösungsmittel i. Vak. abgedampft und das erhaltene Öl mit Methylenchlorid/Methanol 1 : 1 an Kieselgel chromatographiert. Die Hauptfraktion ergibt nach Eindampfen 1.2 g (67 %) die Titelverbindung als zähes, farbloses Öl.

Rf = 0,72 ($CH_3OH/NH_3$ konz. 99 : 1 )

$C_{19}H_{29}N_5O_2$ (359.5)

| | | |
|---|---|---|
| $^1$H-NMR-Spektrum (CDCl$_3$, TMS als interner Standard) | $\delta$ = 1.23 - 1.73 (m) | 6 H, |
| | 2.21 - 2.50 (m) | 4 H, |
| | 2.71 (d) | 3 H, |
| | 3.17 - 3.61 (m) | 4 H, |
| | 3.48 (s) | 2 H, |
| | 3.92 (m) | 1 H, |
| | 4.49 (s) | 2 H, |
| | 4.60 (breit) | 1 H, austauschbar mit $D_2O$ |
| | 6.19 (t) | 1 H, austauschbar mit $D_2O$ |
| | 6.50 (q) | 1 H, austauschbar mit $D_2O$ |
| | 7.27 (s, breit) | 4 H |

Beispiel 8

a) Herstellung von

2-[2-Hydroxy-3-[5-(1-piperidylmethyl)-2-thienylthio]propyl]-1H-isoindol-1,3-dion

Eine Mischung aus 6.84 g (20 mmol) 5-(1-Piperidylmethyl)-2-S-isothioharnstoff-methylthiophen-dihydrochlorid und 6.1 g (30 mmol) N-(2,3-Epoxypropyl) phthalimid wird in 50 ml Ethanol vorgelegt und bei 0 - 5 °C langsam mit

2.4 g (60 mmol) NaOH, gelöst in 60 ml Ethanol, versetzt. Anschließend läßt man 1 h bei 0 - 5 °C, dann 3 h bei Raumtemperatur reagieren. Die Reaktionslösung wird im Vakuum eingeengt, der Rückstand in $CH_2Cl_2$/MeOH (80 : 20) aufgenommen, die organische Phase mit Wasser neutralgewaschen, über $Na_2SO_4$ getrocknet und im Vakuum eingeengt. Man erhält 8.3 g (96 %) der Titelverbindung als braunes Öl.

b) Herstellung von

2-Hydroxy-3-[5-(1-piperidylmethyl)-2-thienylthio] propylamin

8.60 g (20 mmol) 2-[2-Hydroxy-3-[5-(1-piperidylmethyl)-2-thienylthio] propyl]-1H-isoindol-1,3-dion und 3.3 ml Hydrazinhydrat (80 %) werden in 80 ml Ethanol 3 Stunden gekocht. Der nach Einengen der Mischung verbleibende Rückstand wird in 50 ml Wasser aufgenommen, mit 8 ml konz. Salzsäure versetzt und filtriert. Das Filtrat wird mit konz. Natronlauge auf pH 12 eingestellt und mit 3 x 40 ml Methylenchlorid extrahiert. Die organische Phase wird mit $Na_2SO_4$ getrocknet und im Vakuum eingedampft. Man erhält 4.9 g (82 % d.Th.) der Titelverbindung als hellgrünes Öl.

c) Herstellung von

3-[2-Hydroxy-3-[5-(1-piperidylmethyl)-2-thienylthio] propylamino]-4-amino-1,2,5-thiadiazol-1-oxid

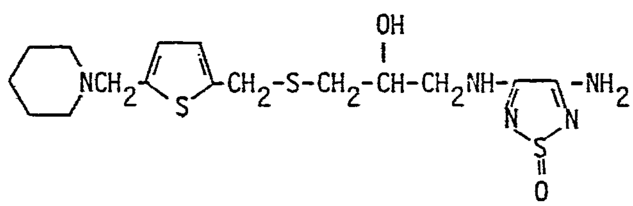

Die Herstellung erfolgt analog Beispiel 1 c,d aus 1.5 g (5mmol) 2-Hydroxy-3-[5-(1-piperidylmethyl)-2-thienylthio] propylamin in 10 ml Ethanol und 0.95 g (5 mmol) 3,4-Diethoxy-1,2,5-thiadiazol-1-oxid.

Farblose Kristalle vom Schmelzpunkt 171 °C

Ausbeute: 1 g (48 % d.Th.)

Rf = 0.25 ($CH_3OH$)

$C_{16}H_{25}N_5O_2S_3$ (415)

| | |
|---|---|
| $^1$H-NMR-Spektrum: (d$_6$-DMSO, TMS als interner Standard) | $\delta$ = 1.20 - 1.57 (m) 6 H, |
| | 2.13 - 2.43 (m) 4 H, |
| | 2.53 (d) 2 H, |
| | 3.13 - 3.50 (m) 2 H, |
| | 3.53 (s) 2 H, |
| | 3.70 - 4.07 (m) 3 H, |
| | 5.23 (s) 1 H, austauschbar mit $D_2O$ |
| | 6.57 - 6.87 (m) 2 H, |
| | 7.70 - 8.23 (m) 3 H, austauschbar mit $D_2O$ |

Beispiel 9

a) Herstellung von

2-[2-Hydroxy-3-[5-(dimethylaminomethyl)-2-furfurylthio]propyl]-1H-isoindol-1,3-dion

Die Herstellung erfolgt analog Beispiel 8 a aus 5-(Dimethylaminomethyl)-2-S-isothioharnstoff-methylfuran-Bismaleat und N-(2,3-Epoxypropyl)-phthalimid.

b) Herstellung von

2-Hydroxy-3-[5-(dimethylaminomethyl)-2-furfurylthio]propylamin

Die Herstellung erfolgt analog Beispiel 8 b.

c) Herstellung von

3-[2-Hydroxy-3-[5-(dimethylaminomethyl)-2-furfurylthio]propylamino]-4-amino-1,2,5-thiadiazol-1-oxid

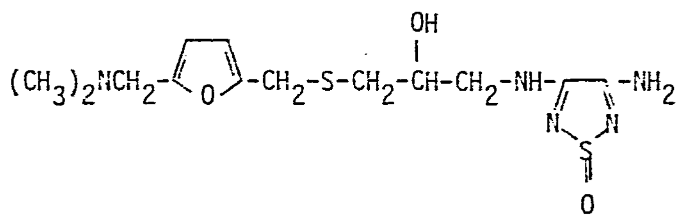

Die Herstellung erfolgt analog Beispiel 8 c.

Farblose Kristalle vom Schmelzpunkt 131 °C

Rf = 0.3 ($CH_3OH$)

| | | | | |
|---|---|---|---|---|
| $C_{13}H_{21}N_5O_3S_2$ (359) | Ber.: | C 43.45 | H 5.85 | N 19.50 |
| | Gef.: | C 43.34 | H 5.77 | N 19.08 |

$^1$H-NMR-Spektrum: ($d_6$-DMSO, TMS als interner Standard)

$\delta$ = 2.13 (s) 6 H,
2.57 (d) 2 H,
3.17 - 3.50 (m) 2 H,
3.77 (m) 3 H,
5.27 (breit) 1 H, austauschbar mit $D_2O$
6.17 (s) 2 H,
7.80 - 8.27 (m) 3 H, austauschbar mit $D_2O$

## Beispiel 10

a) Herstellung von

2-[2-Hydroxy-3-[2-guanidinothiazol-4-yl)methylthio]propyl]-1H-isoindol-1,3-dion

Die Herstellung erfolgt analog Beispiel 8 a aus [2-(Guanidinothiazol-4-yl)-methyl]-S-isothioharnstoff-dihydrochlorid und N-(2,3-Epoxypropyl)-phthalimid.

b) Herstellung von

2-Hydroxy-3-[2-guanidinothiazol-4-yl)methylthio]propylamin

Die Herstellung erfolgt analog Beispiel 8 b.

c) Herstellung von

3-[2-Hydroxy-3-[2-guanidinothiazol-4-yl)methylthio]propylamino]-4-amino-1,2,5-thiadiazol-1-oxid

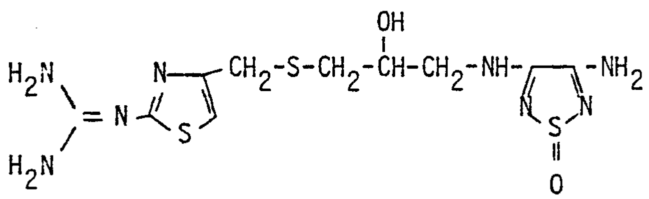

Die Herstellung erfolgt analog Beispiel 8 c.

Farblose Kristalle vom Schmelzpunkt 134 - 135 °C

Rf = 0.8 ($CH_2Cl_2/CH_3OH$ 80 : 20 )

$C_{10}H_{16}N_8O_2S_3$ (376)

| | |
|---|---|
| $^1$H-NMR-Spektrum: | $\delta$ = 2.60 (d) 2 H, |
| ($d_6$-DMSO, TMS als | 3.23 - 4.17 (m) 5 H, |
| interner Standard) | 5.27 (breit) 1 H, austauschbar mit $D_2O$ |
| | 6.50 (s) 1 H, |
| | 6.87 (breit) 4 H, austauschbar mit $D_2O$ |
| | 7.80 - 8.40 (m) 3 H, austauschbar mit $D_2O$ |

0154721

1

LUDWIG HEUMANN & CO · GMBH

8500 Nürnberg

---

Neue Alkanolderivate, Verfahren zu ihrer Herstellung und diese Verbindungen enthaltendes Arzneimittel

---

## PATENTANSPRÜCHE

1. Alkanolderivate der allgemeinen Formel I

$$R^1R^2N-(CH_2)_m-Q-CH_2X-CH_2-Y-(CH_2)_n-NHR^3 \qquad (I)$$

in der im Falle m = 1 $R^1$ und $R^2$, die gleich oder verschieden sein können, jeweils für Wasserstoff, $C_{1-10}$-Alkyl, $C_{5-6}$-Cycloalkyl, Amino, Niedrigalkylamino, Niedrigdialkylamino oder im Falle m = 0 für $(NH_2)_2C=$ stehen oder $R^1$ und $R^2$ zusammen mit dem Stickstoffatom, an das sie gebunden sind, einen 5- bis 8gliedrigen alicyclischen unsubstituierten oder mit einer Methylgruppe substituierten heterocyclischen Ring bilden, m für 0 oder 1 steht und Q einen Furan-, Thiophen-, Thiazol- oder Benzolring bedeutet, X für ein Schwefel- oder Sauerstoffatom oder für die Gruppe -CHOH steht, Y eine Einfachbindung oder die Gruppe -CHOH darstellt, n 1 oder 2 bedeutet und $R^3$ für die Gruppierung

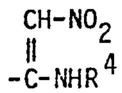, 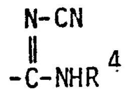, 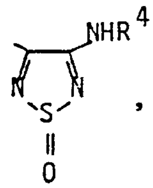, 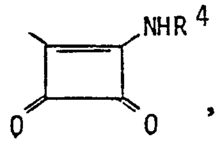,

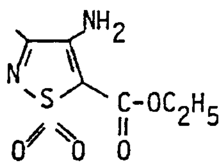 oder 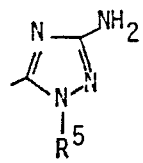

steht, worin $R^4$ ein Wasserstoffatom, eine $C_{1-3}$-Alkylgruppe oder eine Propargylgruppe, $R^5$ ein Wasserstoffatom oder eine $C_{1-3}$-Alkylgruppe bedeutet, sowie ihre physiologisch annehmbaren Salze und Hydrate.

2. Alkanolderivate nach Anspruch 1, dadurch gekennzeichnet, daß m = 0 ist, $R^1$ und $R^2$ jeweils für $(NH_2)_2C=$ steht, Q für einen in 2,4-Stellung eingefügten Thiazolring steht, X für ein Schwefelatom und Y für die Gruppierung -CHOH steht, n = 1 ist und $R^3$ die in Anspruch 1 angegebene Bedeutung besitzt.

3. Alkanolderivate nach Anspruch 1, dadurch gekennzeichnet, daß m = 1 ist, $R^1$ und $R^2$, die gleich oder verschieden sein können, jeweils für $C_{1-3}$-Alkyl oder $C_{5-6}$-Cycloalkyl stehen oder $R^1$ und $R^2$ zusammen mit dem Stickstoffatom, an das sie gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethyleniminooder Octamethyleniminoring bilden, Q für einen in 2,5- oder 2,4-Stellung eingefügten Thiophen- oder einen in 2,5-Stellung eingefügten Furanring steht, X ein Schwefelatom, Y die Gruppierung -CHOH bedeutet, n = 1 ist und $R^3$ die in Anspruch 1 angegebene Bedeutung besitzt.

4. Alkanolderivate nach Anspruch 1, dadurch gekennzeichnet, daß m = 1 ist, $R^1$ und $R^2$, die gleich oder verschieden sein können, jeweils für $C_{1-3}$-Alkyl oder $C_{5-6}$-Cycloalkyl stehen oder $R^1$ und $R^2$

zusammen mit dem Stickstoffatom, an das sie gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring bilden, Q für einen in den Rest des Moleküls durch Bindungen in 1- und 3- oder 1- und 4-Stellung eingefügten Benzolring steht, X ein Sauerstoffatom, Y die Gruppe -CHOH bedeutet, n = 1 ist und $R^3$ die in Anspruch 1 angegebene Bedeutung besitzt.

5. Alkanolderivate nach Anspruch 1, dadurch gekennzeichnet, daß m = 1 ist, $R^1$ und $R^2$, die gleich oder verschieden sein können, jeweils für $C_{1-3}$-Alkyl oder $C_{5-6}$-Cycloalkyl stehen oder $R^1$ und $R^2$ zusammen mit dem Stickstoffatom, an das sie gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring bilden, Q für einen in den Rest des Moleküls durch Bindungen in 1- und 3- oder 1- und 4-Stellung eingefügten Benzolring steht, X die Gruppierung -CHOH bedeutet, Y eine Einfachbindung darstellt, n = 1 ist und $R^3$ die in Anspruch 1 angegebene Bedeutung besitzt.

6. Alkanolderivate nach Anspruch 1, dadurch gekennzeichnet, daß m 1 ist, $R^1$ und $R^2$, die gleich oder verschieden sein können, jeweils für $C_{1-2}$-Alkyl oder $C_{5-6}$-Cycloalkyl stehen oder $R^1$ und $R^2$ zusammen mit dem Stickstoffatom, an das sie gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring bilden, Q für einen in den Rest des Moleküls durch Bindungen in 1- und 3- oder 1- und 4-Stellung eingefügten Benzolring steht, X die Gruppierung -CHOH bedeutet, Y eine Einfachbindung darstellt, n = 2 ist und $R^3$ die in Anspruch 1 angegebene Bedeutung besitzt.

7. 3-[2-Hydroxy-3-[3-(1-piperidylmethyl)-benzyloxy]propylamino]-4-amino-1,2,5-thiadiazol-1-oxid und die physiologisch annehmbaren Salze und Hydrate davon.

8. 1-[2-Hydroxy-3-[3-(1-piperidylmethyl)benzyloxy]propylamino]-2-amino-cyclobuten-3,4-dion und die physiologisch annehmbaren Salze und Hydrate davon.

9. 3-[2-Hydroxy-3-[3-(1-piperidylmethyl)benzyloxy]propylamino]-4-amino-5-ethoxycarbonyl-isothiazol-1,1-dioxid und die physiologisch annehmbaren Salze und Hydrate davon.

10. Verfahren zur Herstellung der Alkanolderivate nach den Ansprüchen 1 bis 9, dadurch g e k e n n z e i c h n e t , daß man

a) zur Herstellung einer Verbindung, bei der $R^3$ für

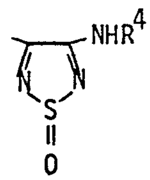 oder 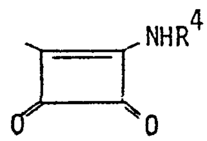

steht, eine Verbindung der allgemeinen Formel II

$$R^1R^2N(CH_2)_m\text{-}Q\text{-}CH_2\text{-}X\text{-}CH_2\text{-}Y\text{-}(CH_2)_nNH\text{-}R^6 \qquad (II)$$

in der $R^1$, $R^2$, m, Q, X, Y und n die in Anspruch 1 angegebenen Bedeutungen besitzen und $R^6$ für die Gruppe

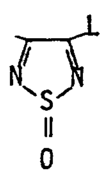 oder 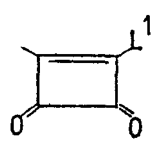

steht, wobei $L^1$ eine Methoxy-, Ethoxy- oder Butoxygruppe als Austrittsgruppe bedeutet, in einem Lösungsmittel mit einem Amin der allgemeinen Formel III

$$R^4NH_2 \qquad (III)$$

in der $R^4$ für ein Wasserstoffatom, eine Methylgruppe oder eine Propargylgruppe steht, zu der erfindungsgemäßen Verbindung der

allgemeinen Formel I umsetzt und die erhaltene Verbindung gegebenenfalls in ihr physiologisch annehmbares Salz umwandelt

oder daß man

b) zur Herstellung einer Verbindung, bei der $R^3$ für $-\overset{\overset{\displaystyle N-CN}{\|}}{C}-NHR^4$ steht, eine Verbindung der allgemeinen Formel IV

$$R^1R^2N(CH_2)_m-Q-CH_2-X-CH_2-Y-(CH_2)_n-NHR^7 \qquad (IV)$$

in der $R^1$, $R^2$, m, Q, X, Y und n die in Anspruch 1 angegebenen Bedeutungen haben und $R^7$ für $\overset{\overset{\displaystyle CH-NO_2}{\|}}{C}-L^2$ oder $-\overset{\overset{\displaystyle N-CN}{\|}}{C}-L^2$ steht, wobei $L^2$ für eine Thiomethyl-, Methoxy-, Ethoxy- oder Phenoxygruppe als Austrittsgruppe steht, mit einem Amin der allgemeinen Formel III

$$R^4NH_2 \qquad (III)$$

zu der erfindungsgemäßen Verbindung der allgemeinen Formel I umsetzt und die erhaltene Verbindung gegebenenfalls in ihr physiologisch annehmbares Salz umwandelt

oder daß man

c) zur Herstellung einer Verbindung, bei der $R^3$ für

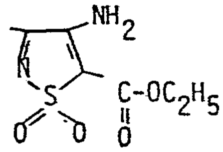

steht, ein Amin der allgemeinen Formel V

$$R^1R^2N(CH_2)_m-Q-CH_2-X-CH_2-Y-(CH_2)_n-NH_2 \qquad (V)$$

in der $R^1$, $R^2$, m, Q, X, Y und n die in Anspruch 1 angegebenen Bedeutungen haben, mit einer Verbindung der Formel VI

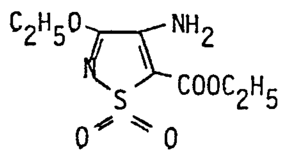

(VI)

zu der erfindungsgemäßen Verbindung der allgemeinen Formel I umsetzt und die erhaltene Verbindung gegebenenfalls in ihr physiologisch annehmbares Salz umwandelt

oder daß man

d) zur Herstellung einer Verbindung, bei der $R^3$ für 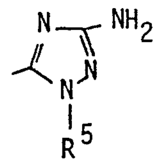

steht und $R^5$ für ein Wasserstoffatom, eine Methyl- oder Ethylgruppe steht, eine Verbindung der allgemeinen Formel VII

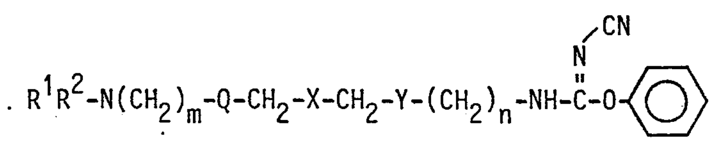

(VII)

in der $R^1$, $R^2$, m, Q, X, Y und n die in Anspruch 1 angegebenen Bedeutungen haben, mit Hydrazinhydrat, Methylhydrazin oder Ethylhydrazin in einem Lösungsmittel zu der erfindungsgemäßen Verbindung der allgemeinen Formel I umsetzt und die erhaltene Verbindung gegebenenfalls in ihr physiologisch annehmbares Salz umwandelt.

11. Arzneimittel, dadurch gekennzeichnet, daß es eine Verbindung nach einem der Ansprüche 1 bis 9 zusammen mit mindestens einem inerten pharmazeutisch annehmbaren Träger oder Verdünnungsmittel enthält.

PATENTANSPRÜCHE für den Vertragsstaat AT:

1. Verfahren zur Herstellung von Alkanolderivaten der allgemeinen Formel I

$$R^1R^2N-(CH_2)_m-Q-CH_2X-CH_2-Y-(CH_2)_n-NHR^3 \qquad (I)$$

in der im Falle m = 1 $R^1$ und $R^2$, die gleich oder verschieden sein können, jeweils für Wasserstoff, $C_{1-10}$-Alkyl, $C_{5-6}$-Cycloalkyl, Amino, Niedrigalkylamino, Niedrigdialkylamino oder im Falle m = 0 für $(NH_2)_2C=$ stehen oder $R^1$ und $R^2$ zusammen mit dem Stickstoffatom, an das sie gebunden sind, einen 5- bis 8gliedrigen alicyclischen unsubstituierten oder mit einer Methylgruppe substituierten heterocyclischen Ring bilden, m für 0 oder 1 steht und Q einen Furan-, Thiophen-, Thiazol- oder Benzolring bedeutet, X für ein Schwefel- oder Sauerstoffatom oder für die Gruppe -CHOH steht, Y eine Einfachbindung oder die Gruppe -CHOH darstellt, n 1 oder 2 bedeutet und $R^3$ für die Gruppierung

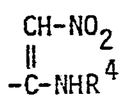 , 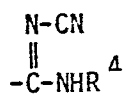 , 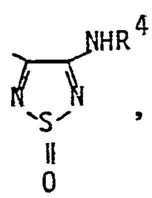 , 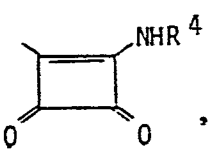 ,

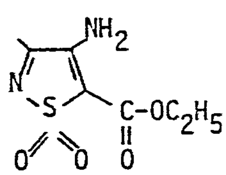 oder 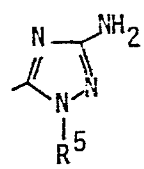

steht, worin $R^4$ ein Wasserstoffatom, eine $C_{1-3}$-Alkylgruppe oder eine Propargylgruppe, $R^5$ ein Wasserstoffatom oder eine $C_{1-3}$-Alkylgruppe bedeutet, sowie ihre physiologisch annehmbaren Salze und Hydrate, dadurch g e k e n n z e i c h n e t , daß man

a) zur Herstellung einer Verbindung, bei der $R^3$ für

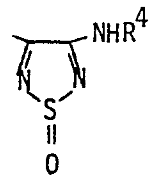 oder 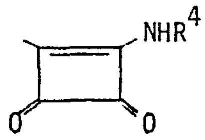

steht, eine Verbindung der allgemeinen Formel II

$$R^1R^2N(CH_2)_m\text{-Q-}CH_2\text{-X-}CH_2\text{-Y-}(CH_2)_n\text{NH-}R^6 \qquad (II)$$

in der $R^1$, $R^2$, m, Q, X, Y und n die oben angegebenen Bedeutungen besitzen und $R^6$ für die Gruppe

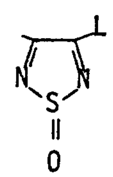 oder 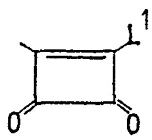

steht, wobei $L^1$ eine Methoxy-, Ethoxy- oder Butoxygruppe als Austrittsgruppe bedeutet, in einem Lösungsmittel mit einem Amin der allgemeinen Formel III

$$R^4NH_2 \qquad (III)$$

in der $R^4$ für ein Wasserstoffatom, eine Methylgruppe oder eine Propargylgruppe steht, zu der erfindungsgemäßen Verbindung der allgemeinen Formel I umsetzt und die erhaltene Verbindung gegebenenfalls in ihr physiologisch annehmbares Salz umwandelt

oder daß man

b) zur Herstellung einer Verbindung, bei der $R^3$ für $\overset{CH-NO_2}{\overset{\|}{-C-NHR^4}}$ oder $\overset{N-CN}{\overset{\|}{-C-NHR^4}}$ steht, eine Verbindung der allgemeinen Formel IV

$$R^1R^2N(CH_2)_m\text{-Q-}CH_2\text{-X-}CH_2\text{-Y-}(CH_2)_n\text{-NHR}^7 \qquad (IV)$$

in der $R^1$, $R^2$, m, Q, X, Y und n die oben angegebenen Bedeutungen haben und $R^7$ für $\overset{CH-NO_2}{\overset{\|}{C-L^2}}$ oder $\overset{N-CN}{\overset{\|}{-C-L^2}}$ steht, wobei $L^2$ für eine

Thiomethyl-, Methoxy-, Ethoxy- oder Phenoxygruppe als Austrittsgruppe steht, mit einem Amin der allgemeinen Formel III

$$R^4NH_2 \qquad (III)$$

zu der erfindungsgemäßen Verbindung der allgemeinen Formel I umsetzt und die erhaltene Verbindung gegebenenfalls in ihr physiologisch annehmbares Salz umwandelt

oder daß man

c) zur Herstellung einer Verbindung, bei der $R^3$ für

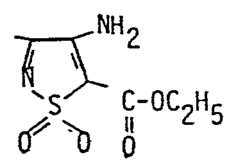

steht, ein Amin der allgemeinen Formel V

$$R^1R^2N(CH_2)_m\text{-}Q\text{-}CH_2\text{-}X\text{-}CH_2\text{-}Y\text{-}(CH_2)_n\text{-}NH_2 \qquad (V)$$

in der $R^1$, $R^2$, m, Q, X, Y und n die oben angegebenen Bedeutungen haben, mit einer Verbindung der Formel VI

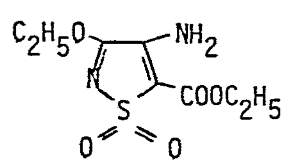

(VI)

zu der erfindungsgemäßen Verbindung der allgemeinen Formel I umsetzt und die erhaltene Verbindung gegebenenfalls in ihr physiologisch annehmbares Salz umwandelt

oder daß man

d) zur Herstellung einer Verbindung, bei der $R^3$ für 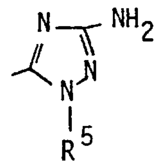 steht und $R^5$ für ein Wasserstoffatom, eine Methyl- oder Ethylgruppe steht, eine Verbindung der allgemeinen Formel VII

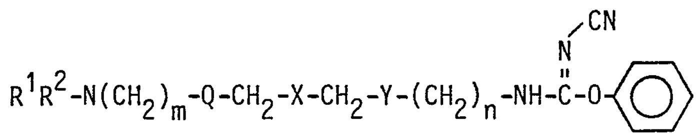 (VII)

in der $R^1$, $R^2$, m, Q, X, Y und n die oben angegebenen Bedeutungen haben, mit Hydrazinhydrat, Methylhydrazin oder Ethylhydrazin in einem Lösungsmittel zu der erfindungsgemäßen Verbindung der allgemeinen Formel I umsetzt und die erhaltene Verbindung gegebenenfalls in ihr physiologisch annehmbares Salz umwandelt.

2. Verfahren nach Anspruch 1 zur Herstellung von Alkanolderivaten, die dadurch gekennzeichnet sind, daß m = 0 ist, $R^1$ und $R^2$ jeweils für $(NH_2)_2C=$ steht, Q für einen in 2,4-Stellung eingefügten Thiazolring steht, X für ein Schwefelatom und Y für die Gruppierung -CHOH steht, n = 1 ist und $R^3$ die in Anspruch 1 angegebene Bedeutung besitzt.

3. Verfahren nach Anspruch 1 zur Herstellung von Alkanolderivaten, die dadurch gekennzeichnet sind, daß m = 1 ist, $R^1$ und $R^2$, die gleich oder verschieden sein können, jeweils für $C_{1-3}$-Alkyl oder $C_{5-6}$-Cycloalkyl stehen oder $R^1$ und $R^2$ zusammen mit dem Stickstoffatom, an das sie gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring bilden, Q für einen in 2,5- oder 2,4-Stellung eingefügten Thiophen- oder einen in 2,5-Stellung eingefügten Furanring steht, X ein Schwefelatom, Y die Gruppierung -CHOH bedeutet, n = 1 ist und $R^3$ die in Anspruch 1 angegebene Bedeutung besitzt.

4. Verfahren nach Anspruch 1 zur Herstellung von Alkanolderivaten, die dadurch gekennzeichnet sind, daß m = 1 ist, $R^1$ und $R^2$, die gleich oder verschieden sein können, jeweils für $C_{1-3}$-Alkyl oder $C_{5-6}$-Cycloalkyl stehen oder $R^1$ und $R^2$ zusammen mit dem Stickstoffatom, an das sie gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring bilden, Q für einen in den Rest des Moleküls durch Bindungen in 1- und 3- oder 1- und 4-Stellung eingefügten Benzolring steht, X ein Sauerstoffatom, Y die Gruppe -CHOH bedeutet, n = 1 ist und $R^3$ die in Anspruch 1 angegebene Bedeutung besitzt.

5. Verfahren nach Anspruch 1 zur Herstellung von Alkanolderivaten, die dadurch gekennzeichnet sind, daß m = 1 ist, $R^1$ und $R^2$, die gleich oder verschieden sein können, jeweils für $C_{1-3}$-Alkyl oder $C_{5-6}$-Cycloalkyl stehen oder $R^1$ und $R^2$ zusammen mit dem Stickstoffatom, an das sie gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring bilden, Q für einen in den Rest des Moleküls durch Bindungen in 1- und 3- oder 1- und 4-Stellung eingefügten Benzolring steht, X die Gruppierung -CHOH bedeutet, Y eine Einfachbindung darstellt, n = 1 ist und $R^3$ die in Anspruch 1 angegebene Bedeutung besitzt.

6. Verfahren nach Anspruch 1 zur Herstellung von Alkanolderivaten, die dadurch gekennzeichnet sind, daß m = 1 ist, $R^1$ und $R^2$, die gleich oder verschieden sein können, jeweils für $C_{1-2}$-Alkyl oder $C_{5-6}$-Cycloalkyl stehen oder $R^1$ und $R^2$ zusammen mit dem Stickstoffatom, an das sie gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring bilden, Q für einen in den Rest des Moleküls durch Bindungen in 1- und 3- oder 1- und 4-Stellung eingefügten Benzolring steht, X die Gruppierung -CHOH bedeutet, Y eine Einfachbindung darstellt, n = 2 ist und $R^3$ die in Anspruch 1 angegebene Bedeutung besitzt.

7. Verfahren nach Anspruch 1 zur Herstellung von 3-[2-Hydroxy-3-[3-(1-piperidylmethyl)-benzyloxy]propylamino]-4-amino-1,2,5-thiadiazol-1-oxid und der physiologisch annehmbaren Salze und Hydrate davon.

8. Verfahren nach Anspruch 1 zur Herstellung von 1-[2-Hydroxy-3-[3-(1-piperidylmethyl)benzyloxy]propylamino]-2-amino-cyclobuten-3,4-dion und der physiologisch annehmbaren Salze und Hydrate davon.

9. Verfahren nach Anspruch 1 zur Herstellung von 3-[2-Hydroxy-3-[3-(1-piperidylmethyl)benzyloxy]propylamino]-4-amino-5-ethoxycarbonyl-isothiazol-1,1-dioxid und der physiologisch annehmbaren Salze und Hydrate davon.

0154721

Europäisches Patentamt

# EUROPÄISCHER RECHERCHENBERICHT

Nummer der Anmeldung

EP 84116345.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | GB - A - 1 601 459 (ALLEN & HANBURYS)<br>* Beispiele 3,4,5; Ansprüche 1, 12b,18 *<br>-- | 1,10,11 | C 07 D 295/08<br>C 07 D 285/10<br>C 07 D 417/12<br>C 07 D 249/14<br>A 61 K 31/40<br>A 61 K 31/41<br>(C 07 D 417/12<br>C 07 D 285:10<br>C 07 D 307:52)<br>C 07 D 417/12<br>C 07 D 285:10<br>C 07 D 277:38) |
| X | GB - A - 2 098 988 (BRISTOL-MYERS)<br>* Beispiele 2-6,20; Anspruch 42 *<br>-- | 1,11 | |
| X | US - A - 4 394 508 (CRENSHAW et al.)<br>* Beispiele 18,24,26,82,84,86, 91,92,109,123,151; Zusammenfassung *<br>-- | 1,11 | |
| X | DE - A1 - 2 917 026 (GLAXO GROUP)<br>* Beispiele 4,7(3,7,8,9,10,17), 35 *<br>-- | 1,11 | RECHERCHIERTE SACHGEBIETE (Int. Cl.4) |
| X | CHEMICAL ABSTRACTS, Band 96, Nummer 10, 8. März 1982, Columbus, Ohio, USA<br>A.A. ALGIERI et al. "1,2,5-Thiadiazole 1-oxide and 1,1-dioxide derivatives. A new class of potent histamine $H_2$-receptor antagonists" Seite 23, Zusammenfassung 79 442j<br>& J. Med. Chem. 1982, 25(3),210-12<br>-- | 1,11 | C 07 D 295/00<br>C 07 D 333/00<br>C 07 D 285/00<br>C 07 D 275/00<br>C 07 D 417/00<br>C 07 D 249/00<br>C 07 D 207/00 |

Der vorliegende Recherchenbericht wurde für alle Patentansprüche erstellt.

| Recherchenort | Abschlußdatum der Recherche | Prüfer |
|---|---|---|
| WIEN | 09-05-1985 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-

EP 84116345.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | CHEMICAL ABSTRACTS, Band 96, Nummer 10, 8. März 1982, Columbus Ohio, USA<br>W.C. LUMMA et al. "Inhibitors of gastric acid secretion: 3,4-diamino-1,2,5-thiadiazole 1-oxides and 1,1 dioxides as urea equivalents in a series of histamine $H_2$ receptor antagonists"<br>Seite 23, Zusammenfassung-Nr. 79 443k<br>& J. Med. Chem. 1982, 25(3), 207-10<br>---- | 1,11 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 09-05-1985 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82